# EUROPÄISCHE PATENTSCHRIFT

(11) **EP 2 671 505 B1**
(45) Veröffentlichungstag und Bekanntmachung des Hinweises auf die Patenterteilung: **02.08.2023**
(21) Anmeldenummer: 13170059.3
(22) Anmeldetag: 31.05.2013
(51) Int. Cl.: A61B 5/021, A61B 5/024, A61B 5/00, A61B 3/107

(54) **Verfahren und Analysegerät zur Messung einer Hornhaut**
Method and analytic device for measuring a cornea
Procédé et appareil d'analyse pour la mesure d'une cornée

(30) Priorität: 08.06.2012 DE 102012209667
(43) Veröffentlichungstag der Anmeldung: 11.12.2013
(73) Patentinhaber: Oculus Optikgeräte GmbH, 35582 Wetzlar (DE)
(72) Erfinder: Steinmüller, Andreas, 35435 Wettenberg (DE)
(74) Vertreter: advotec.

(56) Entgegenhaltungen:
- JP-A- 2004 113 382
- US-A1- 2010 238 408
- US-A1- 2011 237 999

## Beschreibung

Die Erfindung betrifft ein Verfahren und ein Keratometer zur Messung einer Hornhaut an einem zu untersuchenden Auge, insbesondere einer Person, mit einem ophthalmologischen Analysesystem des Keratometers, wobei mit dem ophthalmologischen Analysesystem eine Topographie einer Hornhaut gemessen wird, wobei mit dem ophthalmologischen Analysesystem in einem Messzeitabschnitt eine Reihe von Bilddatensätzen von einem Oberflächenbereich der Hornhaut gewonnen wird, wobei in dem Messzeitabschnitt eine Änderung eines intraokularen Drucks eines Auges durch eine Herz-Kreislaufaktivität erfolgt, wobei durch diese Änderung des intraokularen Drucks eine wiederkehrende Änderung der Topographie der Hornhaut in dem Messzeitabschnitt bewirkt wird, wobei die durch die Herz-Kreislaufaktivität bewirkte, wiederkehrende Änderung der Topographie der Hornhaut aus der Reihe von Bilddatensätzen bestimmt wird, wobei die durch die Herz-Kreislaufaktivität bewirkte, wiederkehrende Änderung der Topographie der Hornhaut gemessen wird, wobei jeweils für bei der Topographiemessung gemessene Punkte des Oberflächenbereichs der Hornhaut die Änderung gemessen wird.

Ophthalmologische Analysesysteme zur Messung einer Topographie einer Hornhaut sind hinreichend bekannt und werden in verschiedenen Varianten, beispielsweise zur Vermessung der Hornhaut oder zur Bestimmung eines Astigmatismus eingesetzt. Weiter können zentrale und periphere Krümmungsradien der Hornhaut gemessen werden, die dann zur Anpassung einer Kontaktlinse verwendet werden können. Sogenannte keratometrische Analysegeräte dienen dabei unter anderem auch zur Bestimmung eines Keratokonus und zur Berechnung einer Intraokularlinse. Durch eine Messung einer Topographie der Hornhaut wird es möglich einen Flächenverlauf einer Hornhautfläche zu beschreiben und so beispielsweise einen Keratokonus zu bestimmen. Insbesondere bei einem Keratokonus besteht das Problem, dass dieser erst nach seiner Entstehung sicher erkannt werden kann. Oft werden in einem Anfangsstadium dieser Augenkrankheit die Symptome zunächst einem Astigmatismus zugeschrieben. So ist eine sichere Bestimmung eines Vorliegens eines Keratokonus nur durch eine Analyse einer Hornhautoberfläche sowie einer Hornhautdicke bzw. Hornhautrückfläche möglich. Eine Möglichkeit zur Früherkennung eines Keratokonus in einer Entstehungsphase, ohne dass Symptome offensichtlich erkennbar sind, ist derzeit nicht bekannt.

Unabhängig von dem vorbeschriebenen keratometrischen Verfahren ist es allgemein bekannt, dass durch eine Herz-Kreislaufaktivität eines Lebewesens bzw. einer Person ein intraokularer Druck eines Auges der Person eine minimale Änderung erfährt. Entsprechend einer durch eine Herzfrequenz bedingten Pulsfrequenz bzw. alternierenden Blutdruckänderung wird ein intraokularer Druck des Auges in im Wesentlichen regelmäßigen Abständen erhöht und gesenkt. Dies wird durch die diesbezügliche Blutdruckerhöhung im Kopfinneren bewirkt, über die auf das Auge eine Kraft ausgeübt wird. Dieser Effekt der Änderung des intraokularen Drucks ist beispielsweise mit einem sogenannten Pascal-Tonometer messbar.

Ein Pascal-Tonometer umfasst einen konkav geformten Tonometerkopf, der an einer Hornhaut zur Anlage gebracht wird sowie einen integrierten, elektronischen Drucksensor, mit dem eine okulare Pulsamplitude bzw. Änderung des intraokularen Drucks in Folge einer Herz-Kreislaufaktivität gemessen werden kann. So können Einflüsse, bedingt durch Eigenschaften der Hornhaut sowie der okularen Pulsamplitude auf die Messung des intraokularen Drucks ausgeschlossen werden, wodurch der intraokulare Druck sehr genau messbar ist. Nachteilig ist, dass es sich hier um ein invasives Messverfahren handelt. Bei einer Messung des intraokularen Drucks mit einem Non-Kontakt-Tonometer wird ein Messergebnis dagegen durch die Herz-Kreislaufaktivität bzw. eine okulare Pulsamplitude beeinflusst. So führt eine Änderung des intraokularen Drucks zu einer ebenfalls minimalen Änderung einer Topographie der Hornhaut. Dieser, ein Messergebnis einer Tonometermessung beeinflussende, störende Effekt der Herz-Kreislaufaktivität wird bei der Messung mit einem Keratometer nicht berücksichtigt, da hier alleine eine Topographie oder Krümmungsradien gemessen werden und die minimale Änderung der Topographie für die Keratometermessung nicht wesentlich ist.

Aus der US 2010/0238408 A1 ist ein mit einem Videokeratometer kombiniertes Non-Contact-Tonometer bekannt, mit dem vor und während einer Verformung einer Hornhaut durch Aufbringen eines Luftstoßes eine Topographieänderung der Hornhaut erfasst beziehungsweise gemessen werden kann. Die Messung eines intraokularen Drucks soll unter anderem auch unter Berücksichtigung von biomechanischen Eigenschaften, wie zum Beispiel einer Elastizität der Hornhaut, erfolgen.

Die JP 2004/113382 beschreibt ein Non-Contact-Tonometer zur Messung eines intraokularen Drucks. Weiter wird ein Puls einer zu untersuchenden Person über eine Kopfauflage des Non-Contact-Tonometers direkt an der Person gemessen, wobei die Messung des intraokularen Drucks mit der Pulsmessung synchronisiert wird, um genauere Messergebnisse zu erhalten.

Aus der US 2011/0237999 A1 ist ein Interferometer bekannt, mit dem eine sogenannte Echtzeitaufnahme einer Hornhaut eines Auges durchgeführt werden kann. Eine Auslenkung der Hornhaut aufgrund einer Herz-Kreislauf-Aktivität wird erfasst und zu einer Differenzkarte mit Spitzen und Tälern für die Herz-Kreislauf-Aktivität verarbeitet. Die Auslenkung der Hornhaut soll als Indikator für eine biomechanische Änderung der Hornhaut infolge eines beispielsweise operativen Eingriffs dienen.

Der vorliegenden Erfindung liegt die Aufgabe zugrunde, ein Verfahren sowie ein Keratometer zur Messung einer Hornhaut vorzuschlagen, mit dem Daten zur verbesserten Identifikation eines Keratokonus bereitgestellt werden.

Diese Aufgabe wird durch ein Verfahren mit den Merkmalen des Anspruchs 1 und ein Analysegerät mit den Merkmalen des Anspruchs 18 gelöst.

Bei dem erfindungsgemäßen Verfahren zur Messung einer Hornhaut an einem zu untersuchenden Auge eines Lebewesens, insbesondere einer Person, mit einem ophthalmologischen Analysesystem eines Keratometers, wird mit dem ophthalmologischen Analysesystem eine Topographie einer Hornhaut gemessen, wobei mit dem ophthalmologischen Analysesystem in einem Messzeitabschnitt eine Reihe von Bilddatensätzen von einem Oberflächenbereich der Hornhaut gewonnen wird, wobei in dem Messzeitabschnitt eine Änderung eines intraokularen Drucks des Auges durch eine Herz-Kreislaufaktivität des Lebewesens bzw. der Person erfolgt, wobei durch diese Änderung des intraokularen Drucks eine wiederkehrende Änderung der Topographie der Hornhaut in dem Messzeitabschnitt bewirkt wird, wobei die durch die Herz-Kreislaufaktivität bewirkte, wiederkehrende Änderung der Topographie der Hornhaut mittels des ophthalmologischen Analysesystems aus der Reihe von Bilddatensätzen bestimmt wird, wobei die durch die Herz-Kreislaufaktivität bewirkte, wiederkehrende Änderung der Topographie der Hornhaut gemessen wird, und wobei jeweils für bei der Topographie gemessene Punkte des Oberflächenbereichs der Hornhaut die Änderung gemessen wird, wobei das ophthalmologische Analysesystem eine Datenbank umfasst, die Daten von Konusstadien verschiedener Personen enthält, wobei ein Vergleich der Messergebnisse mit den gespeicherten Messergebnissen der Personen durchgeführt wird.

Im Rahmen des erfindungsgemäßen Verfahrens wird der sonst unerwünschte Effekt einer wiederkehrenden Änderung der Topographie der Hornhaut durch die Herz-Kreislaufaktivität genutzt, um eine Beschaffenheit der Hornhaut näher zu analysieren. Dabei ist vorgesehen, innerhalb des Messzeitabschnittes eine Reihe von Bilddatensätzen von dem Oberflächenbereich der Hornhaut zu gewinnen. Der Messzeitabschnitt ist dabei zumindest so lang, dass eine Änderung der Topographie der Hornhaut bestimmt bzw. eine Periode gemessen werden kann. Dabei ist es möglich für jeden Bilddatensatz eine jeweilige Topographie der Hornhaut zu messen und je nach Ausbildung des ophthalmologischen Analysesystems diese Messung für eine Vielzahl von Punkten auf einer Hornhautoberfläche durchzuführen. Je nach optischer Auflösung eines jeweiligen, aufgenommenen Bildes der Hornhautoberfläche kann die Topographiemessung für bis zu jeden Bildpunkt der jeweiligen Aufnahme durchgeführt werden. Durch einen Vergleich übereinstimmender Punkte der Hornhautoberfläche aus unterschiedlichen Bilddatensätzen ist es dann möglich, die Änderung bzw. Bewegung des untersuchten Oberflächenbereichs der Hornhaut im Messzeitabschnitt zu messen.

Durch diese Messung lassen sich Rückschlüsse auf eine veränderte Geometrie der Hornhaut ziehen. In Oberflächenbereichen mit beispielsweise einer verminderten Hornhautdicke kommt es bei einer Erhöhung des intraokularen Drucks infolge der Herz-Kreislaufaktivität in dem betreffenden Bereich zu einer verstärkten Auslenkung bzw. Ausstülpung der Hornhaut der durch die Erhöhung des intraokularen Drucks bedingten Änderung der Topographie der Hornhaut. In dem Bereich verminderter Hornhautdicke kann die Hornhaut einer Druckerhöhung nur einen geringeren Widerstand entgegensetzen. Weiter sind Bereiche der Hornhautoberfläche mit abweichenden Materialeigenschaften, zum Beispiel mit einer verminderten Steifigkeit der Hornhaut, so auch messbar. Insgesamt ist es so möglich durch eine nicht invasive Messung der Hornhaut Oberflächenbereiche der Hornhaut zu lokalisieren, die von anderen Oberflächenbreichen abweichende Eigenschaften aufweisen, woraus Rückschlüsse auf eine mögliche Erkrankung der betreffenden Hornhautbereiche gezogen werden können. Eine Erkennung bzw. Bestimmung einer in einem Anfangsstadium befindlichen Hornhauterkrankung wird dadurch wesentlich erleichtert.

Erfindungsgemäß wird mit einem zweiten, beispielsweise kardiologischen Analysesystem eine Herz-Kreislaufaktivität einer zu untersuchenden Person gemessen, wobei dann mit dem zweiten Analysesystem eine Reihe von Messdatensätzen von einer Herz-Kreislauffrequenz der Person in dem Messzeitabschnitt gewonnen wird, wobei die wiederkehrende Veränderung der Topographie der Hornhaut aus der Reihe von Bilddatensätzen mit den Messdaten der Herz-Kreislauffrequenz in dem Messzeitabschnitt synchronisiert wird. Die Herz-Kreislaufaktivität kann mit einer Reihe von Messverfahren bestimmt werden. Es wird parallel zur Messung der Änderung der Topographie der Hornhaut im selben Messzeitabschnitt die Herzkreislauffrequenz der zu untersuchenden Person gemessen und eine Reihe von Messdatensätzen in dem Messzeitabschnitt gewonnen und aufgezeichnet. Die Messdatensätze werden mittels einer Verarbeitungseinrichtung zur Datenverarbeitung mit den Bilddatensätzen des ophthalmologischen Analysesystems synchronisiert. Dabei kann eine Anpassung der Messdatensätze auf die Bilddatensätze oder umgekehrt erfolgen. Die Synchronisation kann dergestalt durchgeführt werden, dass eine Änderung oder eine Amplitude bzw. Periode der Herz-Kreislaufaktivität mit einer Änderung oder Amplitude bzw. Periode der Änderung der Topographie der Hornhaut im Wesentlichen übereinstimmt. Dies ist besonders einfach möglich, da die Änderung der Topographie der Hornhaut von der Herz-Kreislaufaktivität bewirkt wird bzw. abhängig ist. Die Synchronisation hat den Vorteil, dass die Änderung oder Amplitude der Änderung der Topographie der Hornhaut besonders genau bestimmt werden kann und eventuelle andere Einflüsse, die eine Änderung der Topographie der Hornhaut bewirken können, wie zum Beispiel ein Tränenfilm, Blinzeln oder eine veränderte Fixation des Auges, ausgeschlossen bzw. herausgefiltert werden können. In einem mehrere Perioden umfassenden Messzeitabschnitt können dann die jeweiligen durch die Herz-Kreislaufaktivität bedingten Änderungen oder Amplituden der Änderung der Topographie der Hornhaut besonders genau bestimmt werden.

Bereits vor einem Symptom einer exzentrischen Hornhautversteilung können Oberflächenbereiche der Hornhaut ermittelt werden, die aufgrund beispielsweise veränderter Materialeigenschaften zukünftig eine Hornhautversteilung ausbilden können. Erfindungsgemäß umfasst das ophthalmologische Analysesystem eine Datenbank, die Daten von Konusstadien verschiedener Personen enthält. Durch einen Vergleich der Messergebnisse mit den gespeicherten Messergebnissen der Personen wird es dann möglich, eine Wahrscheinlichkeit einer Entstehung eines Keratokonus zu berechnen. Weiter ist es auch möglich, erste Symptome eines Keratokonus aufgrund der Messung der Hornhaut sicher als solche zu identifizieren und beispielsweise nicht mit einem Astigmatismus zu verwechseln.

Vorzugsweise kann eine Amplitude der Änderung der Topographie der Hornhaut gemessen werden, wobei dann jeweils für die bei der Topographiemessung gemessenen Punkte die Amplitude gemessen wird. Bei einer wiederkehrenden Änderung der Topographie der Hornhaut kann eine maximale und/oder minimale Amplitude der Bewegung eines gemessenen Punktes besonders einfach lokalisiert werden. Die betreffenden Amplituden eignen sich daher besonders gut als eine Referenz zur Erkennung einer zyklischen, wiederkehrenden Bewegung der Hornhaut. Weiter können die betreffenden Amplituden als ein Maßstab für die Bewegung der Hornhaut herangezogen werden. Unter einer Amplitude wird hier ein Maximum einer Auslenkung einer Wechselgröße verstanden. Bei dem erfindungsgemäßen Verfahren wird zumindest eine obere bzw. maximale Amplitude der Änderung der Topographie der Hornhaut, das heißt, die Amplitude, die durch eine systolische Herz-Kreislaufaktivität bedingt ist, bestimmt. Unter einer unteren bzw. minimalen Amplitude wird hier folglich die Amplitude verstanden, die durch eine diastolische Herz-Kreislaufaktivität bewirkt wird.

In einer Ausführungsform des Verfahrens kann ein Vergleich von Mittelwerten der jeweiligen wiederkehrenden Änderungen der Punkte durchgeführt werden. So kann der Messzeitabschnitt so gewählt werden, dass eine Vielzahl von wiederkehrenden Änderungen der Topographie der Hornhaut gemessen werden können. Insbesondere durch die Messung der Vielzahl von wiederkehrenden Änderungen kann eine Messgenauigkeit noch weiter verbessert werden, da dann wiederholt auftretende Änderungen des Oberflächenbereichs sicher bestimmt werden können. So ist es möglich eine Periodendauer der Änderung der Topographie der Hornhaut zu messen, wobei für die jeweilige Messung der Periodendauer beispielsweise eine maximale Amplitude, eine minimale Amplitude, ein quadratischer Mittelwert oder ein Effektivwert der jeweiligen Amplitude bzw. Periodendauer bestimmt werden kann. Beispielsweise können dann die Mittelwerte für die gemessenen Punkte des Oberflächenbereichs unter einem maximalen intraokularen bzw. systolischen Druck gemessen werden, um Bereiche des Oberflächenbereichs der Hornhaut zu lokalisieren, in dem die Mittelwerte signifikant von Mittelwerten vergleichbarer Punkte bzw. Bereiche der Hornhaut abweichen. Alternativ ist es auch möglich, die betreffenden Werte ausgehend von beispielsweise einer minimalen Amplitude der Änderung der Topographie der Hornhaut zu berechnen.

In einer weiteren Ausführungsform des Verfahrens kann ein Vergleich der gemessenen Änderung der Topographie der Hornhaut in Relation zu einer vorausgesetzten Änderung der Topographie der Hornhaut für die jeweiligen Punkte durchgeführt werden. Dies kann dadurch erfolgen, dass beispielsweise die gemessene maximale Amplitude für einen Punkt des Oberflächenbereichs der Hornhaut mit einem Wert für eine vorausgesetzte Amplitude verglichen wird. Dies kann für sämtliche gemessenen Punkte des Oberflächenbereichs der Hornhaut durchgeführt werden. Die Vorausgesetzten Werte für die jeweiligen Punkte können beispielsweise einer Datenbank des ophthalmologischen Analysesystems entnommen werden. Der Vergleich kann für sich alleine oder auch in Kombination mit einer Mittelwertbildung der wiederkehrenden Änderungen der Punkte durchgeführt werden.

Weiter ist es dann auch möglich eine Phase der Änderung der Topographie relativ zu einer Phase der Herz-Kreislauffrequenz zu korrigieren. Dies kann beispielsweise in Bezug auf eine absolute Höhe einer Amplitude oder einer Frequenz bzw. Periode erfolgen. Eventuelle Änderungen einer Herz-Kreislaufaktivität während des Messzeitabschnitts können so bei einer Interpretation der Änderung der Topographie der Hornhaut berücksichtigt werden. Dadurch können eventuelle Messfehler noch weiter minimiert werden. Beispielsweise kann ein Messzeitabschnitt eine Länge von bis zu einer Minute haben.

Vorteilhaft kann das zweite Analysesystem ein sphygmologisches Analysesystem sein. Ein sphygmologisches Analysesystem zur Messung eines Pulses ist besonders einfach und kostengünstig erhältlich und kann leicht mit einem ophthalmologischen Analysesystem, beispielsweise in einem gemeinsamen Gehäuse, kombiniert werden. In einer besonders einfachen Variante kann eine Pulsmessung an einem Finger oder einem Ohr einer zu untersuchenden Person erfolgen. Die Person wird dann durch die Pulsmessung kaum beeinträchtigt. Alternativ ist es natürlich auch möglich die Herz-Kreislaufaktivität durch ein anderes bekanntes Verfahren, wie beispielsweise ein Elektrokardiogramm, zu messen.

Besonders vorteilhaft ist es, wenn der systolische, arterielle Puls gemessen wird. Dieser Puls gibt unter anderem Aufschluss über einen absoluten Druck der Gefäße und ist daher für eine Synchronisation mit einer maximalen Amplitude der Topographiemessung besonders geeignet. Alternativ ist es natürlich auch möglich den diastolischen Puls zu messen.

Auch kann vorgesehen sein, dass eine Blutdruckänderung kontinuierlich gemessen wird. Dann ist es möglich einen Puls bzw. die Blutdruckänderung kontinuierlich zu überwachen, wodurch eine Bestimmung des systolischen und/oder diastolischen Pulses besonders einfach möglich wird. Auch können dann bestimmte, wiederkehrende Zeitabschnitte der Blutdruckänderung für eine Messung bzw. die Synchronisation der wiederkehrenden Änderung der Topographie der Hornhaut herangezogen werden. Weiter kann die kontinuierliche Messung der Blutdruckänderung auch für eine spätere Auswertung gespeichert werden.

Weiter kann die Änderung der Topographie zwischen einem Anfang und einem Ende der Systole gemessen werden. Bezogen auf eine Blutdruckänderung bedeutet dies, dass im Bereich eines Anstiegs des Blutdrucks, also an einem Ende der Diastole bzw. Anfang der Systole, und einer maximalen Amplitude bzw. im Bereich eines maximalen Werts des Blutdrucks der betreffenden Systole, also einem Ende der Systole bzw. Anfang der Distole, eine Änderung der Topographie in den jeweiligen Punkten der Hornhaut gemessen werden kann. Durch den Bezug der Messung auf den vorgenannten Abschnitt der Systole wird es dann möglich, eine Bewegungsänderung der Topographie innerhalb des betreffenden Zeitraums, zwischen dem Anfang und dem Ende der Systole, zu messen. Die Bewegungsänderung kann beispielsweise in einem Weg-Zeit-Diagramm dargestellt werden, wobei für die Änderung der Topographie zwischen dem Anfang und dem Ende der Systole jeweils eine Steigung einer Kurve der Bewegungsänderung ermittelt werden kann.

Noch genauere Messergebnisse können erzielt werden, wenn die Änderung der Topographie zwischen einer minimalen Pulsamplitude und einer maximalen Pulsamplitude gemessen wird. Diese Pulsamplituden können vergleichsweise verlässlich bestimmt werden.

Aus der Änderung der Topographie der Hornhaut kann weiter eine Krümmungsänderung ΔK der Hornhaut abgeleitet werden. Eine Krümmungsänderung kann sich insbesondere dadurch ergeben, dass ein intraokularer Druck, wie vorbeschrieben durch die Herz-Kreislaufaktivität, erhöht wird. So kann dann nicht alleine eine Relativbewegung eines Punktes der Oberfläche der Hornhaut gemessen, sondern gleichzeitig bestimmt werden, welche Krümmungsänderungen ΔK der Hornhaut sich aus der Bewegung des Punktes ergeben. Dazu ist es dann erforderlich, dass eine Mehrzahl von Punkten der Oberfläche der Hornhaut gleichzeitig gemessen wird.

Optional kann eine Relativbewegung des Oberflächenbereichs der Hornhaut in Richtung einer Längsachse des untersuchten Auges von zumindest 0,01 mm gemessen werden. Breits mit dieser Messgenauigkeit wird es möglich das Verfahren zur Messung der Hornhaut durchzuführen. Diese Messgenauigkeit kann beispielsweise erzielt werden, wenn als ophthalmologisches Analysegerät ein Keratometer verwendet wird.

Vorteilhaft kann über den Messzeitabschnitt ein Mittelwert für die jeweiligen Krümmungsänderungen ΔK der Punkte gebildet werden. Für den Fall, dass die Änderung der Topographie der Hornhaut nicht mit der Herz-Kreislaufaktivität synchronisiert wäre, würde der Mittelwert über einen längeren Messzeitabschnitt den Wert Null annehmen, da dann hier nahezu alle Abschnitte einer Kurve einer Bewegung eines Punktes in einem Weg-Zeit-Diagramm berücksichtigt werden würden. Durch eine Synchronisation mit der Herz-Kreislaufaktivität bzw. einer Blutdruckänderung und einer Messung der Krümmungsänderung ΔK in durch die Blutdruckänderung festgelegten Abschnitten, beispielsweise jeweils einem Anstiegsbereich einer Systole, führt eine Mittelwertbildung einer Krümmungsänderung ΔK dann stets zu einem positiven Wert. Diese Werte sind dann auch für die gemessenen Punkte des Oberflächenbereichs der Hornhaut vergleichbar, derart, dass Oberflächenbereiche der Hornhaut einen höheren oder niedrigeren Mittelwert einer Krümmungsänderung ΔK aufweisen können. Aus derartigen Messergebnissen können dann besonders verlässliche Angaben über eine Beschaffenheit eines Hornhautmaterials abgeleitet werden.

Als eine Materialeigenschaft der Hornhaut, die zur Bestimmung eines Keratokonus herangezogen werden kann, kann eine Steifigkeit der Hornhaut für jeweils die gemessenen Punkte des Oberflächenbereichs der Hornhaut bestimmt werden, wobei die Steifigkeit der Hornhaut aus einer Steigung der Krümmungsänderung ΔK angeleitet werden kann. So kann eine Verteilung der Steifigkeit der Hornhaut über den gemessenen Oberflächenbereich als ein Messergebnis erhalten werden, woraus wiederum Rückschlüsse auf eine Erkrankung der Hornhaut bzw. des Auges gezogen werden können. Beispielsweise lässt eine besonders starke Krümmungsänderung ΔK in einem Hornhautbereich gegenüber sonst niedrigen Krümmungsänderungen ΔK in den übrigen Hornhautbereichen den Schluss zu, dass der Hornhautbereich mit der starken bzw. hohen Krümmungsänderung besonders flexibel und damit wenig steif ist. Unter einer Steifigkeit wird hier der Widerstand des Hornhautmaterials gegen eine Verformung durch eine Kraft verstanden. Die Steifigkeit der Hornhaut hängt von der Elastizität des Hornhautmaterials bzw. des Elastizitätsmoduls oder des Schubmoduls, aber auch von der Größe und Form der betreffenden Querschnittsfläche der Hornhaut ab.

Eine zu untersuchende Person wird bei der Messung der Hornhaut besonders wenig beeinträchtigt, wenn eine nicht invasive Messung der Herz-Kreislauffrequenz durchgeführt wird. Eine Messung eines Pulses an einem Finger oder einem Ohr entspricht im Wesentlichen einer nicht invasiven Messung. Grundsätzlich wird mit der nicht invasiven Messung der Herz-Kreislauffrequenz das Ziel verfolgt, einen okularen Pulszyklus zu messen. Zwar kann dies auch durch eine invasive Messung direkt am zu untersuchenden Auge erfolgen, dies wird jedoch von den betreffenden Patienten bzw. Personen als unangenehm empfunden.

Weiter kann mit dem ophthalmologischen Analysesystem aus den Bilddatensätzen eine Messung eines Tränenfilms in dem Messzeitabschnitt durchgeführt werden. Wenn der Messzeitabschnitt so lang gewählt wird, dass der vorhandene Tränenfilm in dem mit dem ophthalmologischen Analysesystem aufgenommenen Oberflächenbereich der Hornhaut eine Änderung erfährt, und zum Beispiel aufreißt, kann die Topographiemessung des Oberflächenbereichs unter anderem verfälscht werden. Zwar erfolgt eine Änderung des Tränenfilms im Vergleich zu einer Herz-Kreislaufaktivität und damit zu einer davon abhängigen Änderung der Topographie der Hornhaut vergleichsweise langsam, und ist damit leicht zu erkennen. Weiter ist eine Herz-Kreislauf-bedingte Änderung der Topographie der Hornhaut kleiner als eine Änderung der Topographie, die durch eine Tränenfilmveränderung bedingt ist. Die Änderung oder die Amplitude der Änderung der Topographie der Hornhaut kann daher nach den Messdaten des Tränenfilms bzw. einer Änderung des Tränenfilms leicht korrigiert werden. Dadurch kann die Messung der Hornhaut noch genauer durchgeführt werden.

Zur besseren Veranschaulichung und zur Auswertung durch eine Bedienperson können die Messergebnisse der Änderungen der Punkte als eine Karte einer Hornhautoberfläche grafisch dargestellt werden. Die grafische Darstellung kann beispielsweise Isolinien oder eine Farbverlaufsdarstellung zur Kennzeichnung der Isolinien aufweisen. Weiter sind perspektivische Darstellungen sowie Darstellungen von Abweichungen in einem Oberflächenbereich eines Auges möglich. Die Darstellung der Messergebnisse kann durch das ophthalmologische Analysesystem erfolgen, sodass unmittelbar nach einer Messung einer Hornhaut einer Bedienperson ein Messergebnis unmittelbar ersichtlich ist.

Das erfindungsgemäße Keratometer zur Messung einer Hornhaut an einem zu untersuchenden Auge, insbesondere einer Person, umfasst ein ophthalmologisches Analysesystem, wobei das ophthalmologische Analysesystem zur Messung einer Topographie einer Hornhaut dient, wobei das ophthalmologische Analysesystem so ausgebildet ist, dass in einem Messzeitabschnitt eine Reihe von Bilddatensätzen von einem Oberflächenbereich der Hornhaut gewonnen werden können, wobei dann in dem Messzeitabschnitt eine Änderung eines intraokularen Drucks des Auges durch eine Herz-Kreislaufaktivität erfolgt, und durch diese Änderung des intraokularen Drucks eine wiederkehrende Änderung der Topographie der Hornhaut in dem Messzeitabschnitt bewirkt wird, wobei das ophthalmologische Analysesystem eine Verarbeitungseinrichtung umfasst, mittels der die durch die Herz-Kreislaufaktivität bewirkte, wiederkehrende Änderung der Topographie der Hornhaut aus der Reihe von Bilddatensätzen bestimmbar ist, wobei mittels der Verarbeitungseinrichtung die durch die Herz-Kreislaufaktivität bewirkte, wiederkehrende Änderung der Topographie der Hornhaut messbar ist, und wobei jeweils für bei der Topographiemessung gemessene Punkte des Oberflächenbereichs der Hornhaut die Änderung messbar ist, wobei das ophthalmologische Analysesystem eine Datenbank umfasst, die Daten von Konusstadien verschiedener Personen enthält, wobei ein Vergleich der Messergebnisse mit den gespeicherten Messergebnissen der Personen durchführbar ist.

Die vorteilhaften Wirkungen des ophthalmologischen Analysegeräts betreffend wird auf die Beschreibung des erfindungsgemäßen Verfahrens verwiesen.

Erfindungsgemäß ist das ophthalmologische Analysegerät ein Keratometer . Mit einem Keratometer kann eine Topographie einer Hornhaut besonders einfach gemessen und bestimmt werden.

Weitere mögliche Ausführungsformen des ophthalmologischen Analysegerätes ergeben sich aus den Merkmalsbeschreibungen der auf den Verfahrensanspruch 1 rückbezogenen Unteransprüche.

Die Erfindung wird durch die Ansprüche definiert.

Nachfolgend wird eine bevorzugte Ausführungsform der Erfindung unter Bezugnahme auf die beigefügten Zeichnungen näher erläutert.

Es zeigen:
- **Fig. 1**: Eine Diagrammdarstellung einer Herz-Kreislaufaktivität und einer Bewegungsänderung eines Punktes einer Hornhaut;
- **Fig. 2**: eine schematische Schnittansicht der Hornhaut eines Auges;
- **Fig. 3**: eine Diagrammdarstellung eines Oberflächenbereichs einer Hornhaut;
- **Fig. 4**: eine weitere Diagrammdarstellung einer Herz-Kreislaufaktivität;
- **Fig. 5**: eine weitere Diagrammdarstellung einer Bewegungsänderung eines Punktes einer Hornhaut.

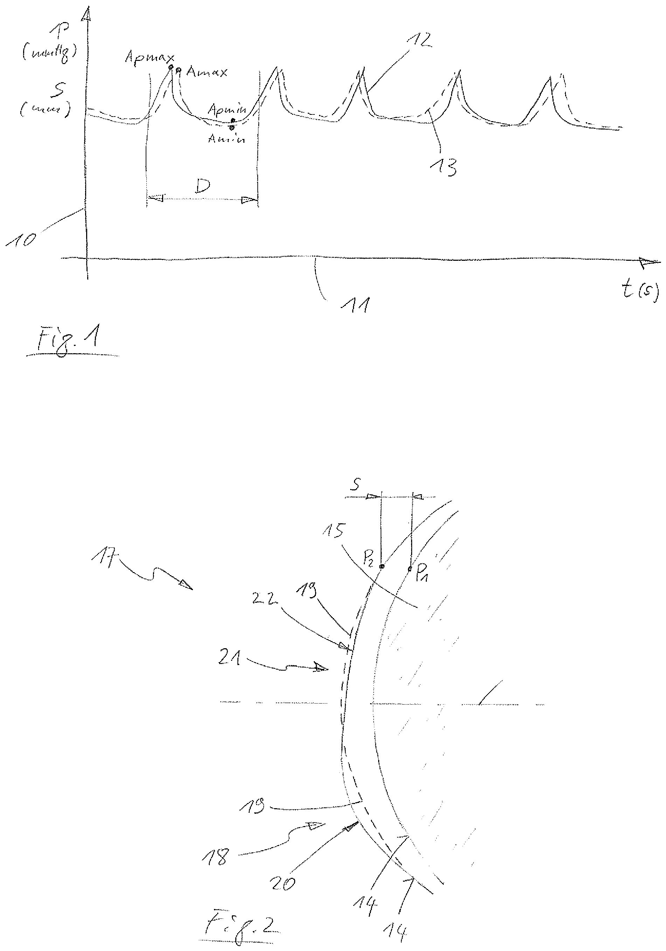

**Fig. 1** zeigt eine Diagrammdarstellung einer Änderung einer Herz-Kreislaufaktivität bzw. eines Pulses einer Person. Auf einer Ordinatenachse 10 ist ein Druck in mmHg und auf einer Abszissenachse 11 eine Zeit in Sekunden einer wiederkehrenden Änderung des Pulses in Form einer wellenförmigen, durchgezogenen Linie 12 dargestellt. Weiter ist auf der Ordinatenachse 10 eine Strecke S in mm für eine wellenförmige, gestrichelte Linie 13 dargestellt. Der abgebildete Zeitabschnitt entspricht hier einem Messzeitabschnitt einer Messung. Die Strecke S ist auf einem Punkt P auf einer Oberfläche 14 einer Hornhaut 15 bezogen, wobei die Linie 13 einen Versatz bzw. eine Bewegung des Punktes P in Richtung einer optischen Achse 16 eines Auges 17 darstellt. Die Linie 13 entspricht hier zu verbesserten Veranschaulichung eine im wesentlichen idealisierte Darstellung einer Bewegung ohne die Bewegung verfälschende Einflüsse, wie zum Beispiel eine Tränenfilmänderung, Blinzeln oder eine Fixationsänderung bzw. Augenbewegung.

Wie in **Fig. 2** gezeigt, wird durch eine Erhöhung eines intraokularen Drucks des hier nur abschnittsweise dargestellten Auges 17 die Oberfläche 14 der Hornhaut 15 im Wesentlichen in Richtung der optischen Achse 16 derart bewegt, dass sich der Punkt P von einer Position P1 um die Strecke S in eine Position P2 bewegt. Diese Bewegung des Punktes P sowie eine Bewegung von übrigen, die Oberfläche 14 der Hornhaut beschreibenden, hier nicht näher bezeichneten Punkten, wird von einem hier ebenfalls nicht näher dargestellten Keratometer während des Messzeitabschnitts dadurch gemessen, dass eine Reihe von Bilddatensätzen aufgenommen wird. So wird es möglich eine Änderung einer Topographie der Hornhaut 15 für eine Vielzahl von Punkten P zu messen.

Wie beispielhaft an einer Periodendauer D der wiederkehrenden Änderung des Punktes P der Topographie zu entnehmen ist, befindet sich der Punkt P in der Position P1 an einer minimalen Amplitude Aₘᵢₙ und in der Position P2 an einer maximalen Amplitude Aₘₐₓ der Linie 13. Die Linie 13, die die Auslenkung des Punktes P relativ zum Messzeitabschnitt darstellt, ist dabei mit der Linie 12, die einen Puls bzw. eine Blutdruckänderung relativ zum Messzeitabschnitt darstellt, synchronisiert. Die Linie 12 gelangt daher mit einer maximalen Amplitude Apₘₐₓ und einer minimalen Amplitude Apₘᵢₙ eines Pulses weitestgehend mit der Linie 13 in Überdeckung.

In **Fig. 2** ist weiter ein Oberflächenbereich 18 der Hornhaut 15 dargestellt, der während einer maximalen Auslenkung der Hornhaut 15 gegenüber einer vorausgesetzten, hier mit einer gestrichelten Linie 19 dargestellten Auslenkung in dem Oberflächenbereich 18 ausgestülpt ist bzw. eine relativ zu der optischen Achse 16 exzentrische Hornhautversteilung aufweist. Diese Ausstülpung 20 ist durch eine Schwächung eines Hornhautgewebes im Oberflächenbereich 18 bedingt und ein erstes signifikantes Anzeichen für eine Ausbildung eines Keratokonus in dem Oberflächenbreich 18. Weiter ist aufgrund der Ausstülpung 20 die Hornhaut 15 in einem Oberflächenbereich 21 abgeflacht bzw. bildet eine Abflachung 22 relativ zu der vorausgesetzten Auslenkung bzw. Linie 19 aus.

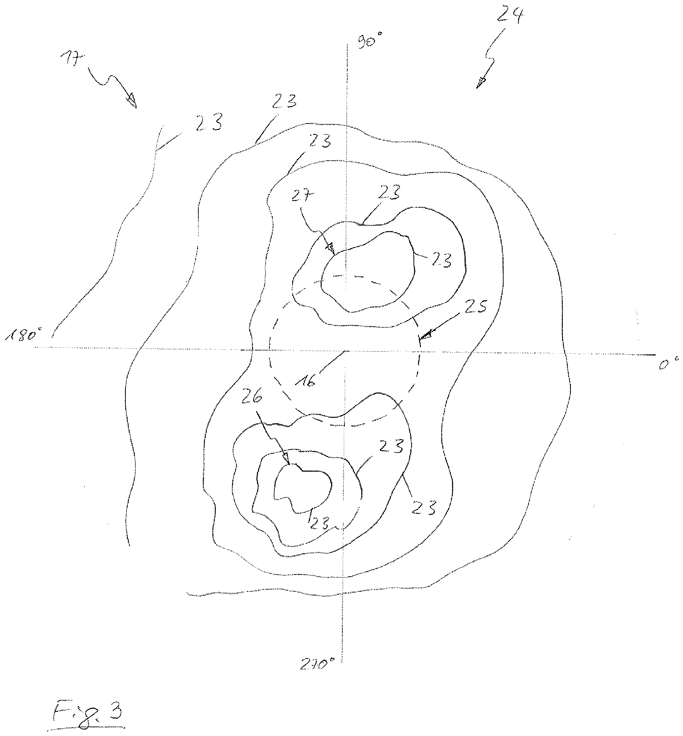

**Fig. 3** zeigt eine Diagrammdarstellung der Hornhaut 15 mit Isolinien 23, die jeweils unterschiedliche Größen eines Mittelwertes für die jeweiligen gemessenen Punkte der Hornhaut 15 darstellen. Die Diagrammdarstellung umfasst einen im Wesentlichen gesamten Oberflächenbereich 24 der Hornhaut 15, der einem Messbereich des Keratometers entspricht. Koaxial zur optischen Achse 16 ist eine Pupille 25 angedeutet. Aus den mit den Isolinien 23 dargestellten unterschiedlichen Mittelwerten der jeweiligen Amplituden der Änderung der Topographie der Hornhaut 15 kann aus der hier gezeigten, beispielhaften Darstellung entnommen werden, dass in einem Oberflächenbereich 26 vergleichsweise hohe Mittelwerte gemessen werden. Diese Mittelwerte sind durch eine stärkere Auslenkung der Hornhaut 15 in dem Oberflächenbereich 24 bedingt, wodurch auf einen entstehenden Keratokonus geschlossen werden kann. Weiter ergibt sich in einem Oberflächebereich 27 analog zum Beispiel nach der **Fig. 2** eine verminderte Auslenkung der Hornhaut 15, was sich in vergleichsweise niedrigen Mittelwerten niederschlägt. Hieraus kann auf eine Abflachung in dem Oberflächenbreich 27 geschlossen werden.

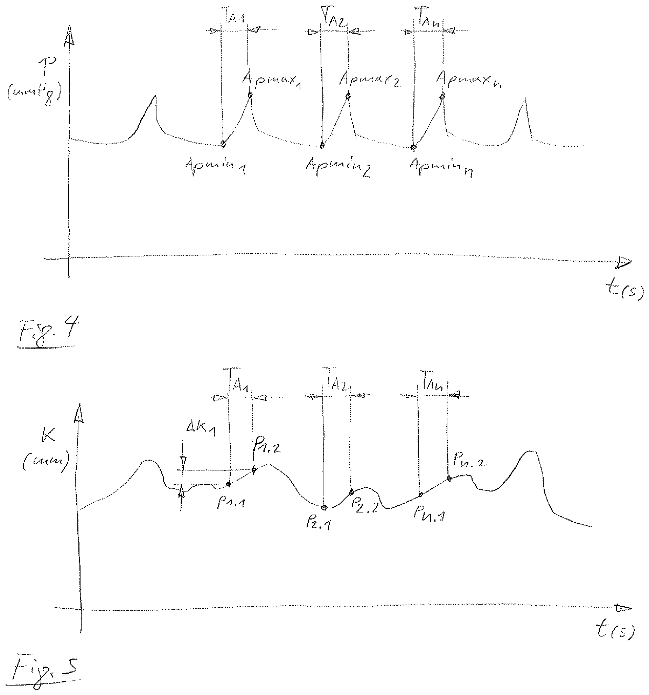

Eine Zusammenschau der **Fig. 4** und **5** zeigt, wie schon in **Fig. 1****,** Diagrammdarstellungen einer Änderung einer Herz-Kreislaufaktivität bzw. eine Krümmungsänderung ΔK. Nach der **Fig. 4** erfolgt eine kontinuierliche Blutdruckmessung, wobei hier jeweils die maximalen und minimalen Amplituden bzw. die Anfangs- und Endpunkte einer Systole ermittelt werden. Nach einer Synchronisation mit einer Änderung der Topographie der Hornhaut erfolgt eine Messung einer Krümmungsänderung ΔK in dem betreffenden, übereinstimmenden Zeitabschnitt T_{A}. Das Diagramm in **Fig. 5** zeigt eine sich ändernde Krümmungsänderung ΔK bezogen auf den dargestellten Messzeitabschnitt, bei dem die Krümmungsänderung nicht ideal verläuft, sondern weiteren Einflüssen, beispielsweise einer Änderung eines Tränenfilms, durch Blinzeln oder einer Änderung einer Fixation des Auges, unterworfen ist. Für den Punkt Apₘᵢₙ₁ im Diagramm der Herz-Kreislaufaktivität wird daher zeitgleich die Krümmungsänderung im Punkt P_{1.1} im Diagramm der Krümmungsänderung bestimmt und nachfolgend für den Punkt Apₘₐₓ₁ die Krümmungsänderung im Punkt P_{1.2}. Hieraus lässt sich eine Steigung der Krümmunsänderung zwischen dem Punkt P_{1.1} und dem Punkt P_{1.2} bestimmen. Diese Steigung der Krümmungsänderung wird für die jeweiligen Zeitabschnitte T_{A1}, T_{A2} bis T_{An} ermittelt und ein Mittelwert der Krümmungsänderung gebildet. Der Mittelwert der Krümmungsänderung bildet somit eine vergleichbare Messgröße für sämtliche gemessenen Punkte P des Oberflächenbereichs der Hornhaut. Insbesondere wird es so möglich, die durch die Herz-Kreislaufaktivität und sonstigen Einflüssen bedingte Änderung der Topographie der Hornhaut bzw. des hier dargestellten Punktes P genau zu analysieren und gegebenenfalls diese Änderung überlagernde, durch andere Einflüsse bewirkte Änderungen herauszufiltern.

## Patentansprüche

1. Verfahren zur Messung einer Hornhaut an einem zu untersuchenden Auge (17), insbesondere einer Person, mit einem ophthalmologischen Analysesystem eines Keratometers, wobei mit dem ophthalmologischen Analysesystem eine Topographie einer Hornhaut (15) gemessen wird, wobei mit dem ophthalmologischen Analysesystem in einem Messzeitabschnitt eine Reihe von Bilddatensätzen von einem Oberflächenbereich (24) der Hornhaut gewonnen wird, wobei in dem Messzeitabschnitt eine Änderung eines intraokularen Drucks des Auges durch eine Herz-Kreislaufaktivität erfolgt, wobei durch diese Änderung des intraokularen Drucks eine wiederkehrende Änderung der Topographie der Hornhaut in dem Messzeitabschnitt bewirkt wird, wobei die durch die Herz-Kreislaufaktivität bewirkte, wiederkehrende Änderung der Topographie der Hornhaut aus der Reihe von Bilddatensätzen bestimmt wird, wobei die durch die Herz-Kreislaufaktivität bewirkte, wiederkehrende Änderung der Topographie der Hornhaut gemessen wird, wobei jeweils für bei der Topographiemessung gemessene Punkte (P) des Oberflächenbereichs der Hornhaut die Änderung gemessen wird,
**dadurch gekennzeichnet,**
**dass** das ophthalmologische Analysesystem eine Datenbank umfasst, die Daten von Konusstadien verschiedener Personen enthält, wobei ein Vergleich der Messergebnisse mit den gespeicherten Messergebnissen der Personen durchgeführt wird.

2. Verfahren nach Anspruch 1,
**dadurch gekennzeichnet,**
**dass** eine Amplitude der Änderung der Topographie der Hornhaut (15) gemessen wird, wobei jeweils für die bei der Topographiemessung gemessenen Punkte (P) die Amplitude gemessen wird.

3. Verfahren nach Anspruch 1 oder 2,
**dadurch gekennzeichnet,**
**dass** ein Vergleich von Mittelwerten der jeweiligen wiederkehrenden Änderungen der Punkte (P) durchgeführt wird.

4. Verfahren nach einem der vorangehenden Ansprüche,
**dadurch gekennzeichnet,**
**dass** ein Vergleich der gemessenen Änderung der Topographie der Hornhaut (15) in Relation zu einer vorausgesetzten Änderung der Topographie der Hornhaut für die jeweiligen Punkte (P) durchgeführt wird.

5. Verfahren nach einem der vorangehenden Ansprüche,
**dadurch gekennzeichnet,**
**dass** einem zweiten Analysesystem eine Herz-Kreislaufaktivität einer zu untersuchenden Person gemessen wird, wobei mit dem zweiten Analysesystem eine Reihe von Messdatendatensätzen von einer Herz-Kreislauffrequenz in dem Messzeitabschnitt gewonnen wird, wobei die wiederkehrende Änderung der Topographie der Hornhaut (15) mit den Messdaten der Herz-Kreislauffrequenz in dem Messzeitabschnitt synchronisiert wird.

6. Verfahren nach Anspruch 5,
**dadurch gekennzeichnet,**
**dass** eine Phase der Änderung der Topographie relativ zu einer Phase der Änderung der Herz-Kreislauffrequenz korrigiert wird.

7. Verfahren nach Anspruch 5 oder 6,
**dadurch gekennzeichnet,**
**dass** das zweite Analysesystem ein sphygmologisches Analysesystem ist.

8. Verfahren nach einem der Ansprüche 5 bis 7,
**dadurch gekennzeichnet,**
**dass** der systolische, arterielle Puls gemessen wird.

9. Verfahren nach einem der Ansprüche 5 bis 8,
**dadurch gekennzeichnet,**
**dass** eine Blutdruckänderung kuntinuierlich gemessen wird.

10. Verfahren nach einem der Ansprüche 5 bis 9,
**dadurch gekennzeichnet,**
**dass** die Änderung der Topographie zwischen einem Anfang und einem Ende der Systole gemessen wird.

11. Verfahren nach einem der Ansprüche 5 bis 10,
**dadurch gekennzeichnet,**
**dass** die Änderung der Topographie zwischen einer minimalen Pulsamplitude und einer maximalen Pulsamplitude gemessen wird.

12. Verfahren nach Anspruch 10 oder 11,
**dadurch gekennzeichnet,**
**dass** aus der Änderung der Topographie der Hornhaut (15) eine Krümmungsänderung (ΔK) der Hornhaut abgeleitet wird.

13. Verfahren nach Anspruch 12,
**dadurch gekennzeichnet,**
**dass** über den Messzeitabschnitt ein Mittelwert für die jeweiligen Krümmungsänderungen (ΔK) der Punkte (P) gebildet wird.

14. Verfahren nach Anspruch 12 oder 13,
**dadurch gekennzeichnet,**
**dass** eine Steigung der Krümmungsänderung (ΔK) abgeleitet wird, wobei aus der Steigung eine Steifigkeit der Hornhaut (15) für jeweils die gemessenen Punkte (P) des Oberflächenbereichs (24) der Hornhaut bestimmt wird.

15. Verfahren nach einem der Ansprüche 5 bis 14,
**dadurch gekennzeichnet,**
**dass** eine nicht invasive Messung der Herz-Kreislauffrequenz durchgeführt wird.

16. Verfahren nach einem der vorangehenden Ansprüche,
**dadurch gekennzeichnet,**
**dass** mit dem ophthalmologischen Analysesystem aus den Bilddatensätzen eine Messung eines Tränenfilms im Messzeitabschnitt durchgeführt wird, wobei die Änderung der Topographie nach den Messdaten des Tränenfilms korrigiert wird.

17. Verfahren nach einem der vorangehenden Ansprüche,
**dadurch gekennzeichnet,**
**dass** die Messergebnisse der Änderungen der Punkte (P) als eine Karte einer Hornhautoberfläche (14) grafisch dargestellt werden.

18. Keratometer zur Messung einer Hornhaut an einem zu untersuchenden Auge (17), insbesondere einer Person, mit einem ophthalmologischen Analysesystem, wobei das ophthalmologische Analysesystem zur Messung einer Topographie einer Hornhaut (15) dient, wobei das ophthalmologische Analysesystem so ausgebildet ist, dass in einem Messzeitabschnitt eine Reihe von Bilddatensätzen von einem Oberflächenbereich (24) der Hornhaut gewonnen werden kann, wobei dann in dem Messzeitabschnitt eine Änderung eines intraokularen Drucks des Auges durch eine Herz-Kreislaufaktivität erfolgt, und durch diese Änderung des intraokularen Drucks eine wiederkehrende Änderung der Topographie der Hornhaut in dem Messzeitabschnitt bewirkt wird, wobei das ophthalmologische Analysesystem eine Verarbeitungseinrichtung umfasst, mittels der die durch die Herz-Kreislaufaktivität bewirkte, wiederkehrende Änderung der Topographie der Hornhaut aus der Reihe von Bilddatensätzen bestimmbar ist, wobei mittels der Verarbeitungseinrichtung die durch die Herz-Kreislaufaktivität bewirkte, wiederkehrende Änderung der Topographie der Hornhaut messbar ist, und wobei jeweils für bei der Topographiemessung gemessene Punkte (P) des Oberflächenbereichs der Hornhaut die Änderung messbar ist,
**dadurch gekennzeichnet,**
**dass** das ophthalmologische Analysesystem eine Datenbank umfasst, die Daten von Konusstadien verschiedener Personen enthält, wobei ein Vergleich der Messergebnisse mit den gespeicherten Messergebnissen der Personen durchführbar ist.

## Claims

1. A method for measuring a cornea of an eye (17) to be examined, in particular of a person, with an ophthalmological analysis system of a keratometer, wherein, with the ophthalmological analysis system, a topography of a cornea (15) is measured, wherein, with the ophthalmological analysis system, in a measurement time interval, a number of image data sets of a surface area (24) of the cornea is obtained, wherein, in the measurement time interval, a change in an intraocular pressure of the eye is effected by a cardiovascular activity, wherein, due to said change in the intraocular pressure, a repeated change in the topography of the cornea in the measurement time interval is caused, wherein the repeated change in the topography of the cornea, which is caused by the cardiovascular activity, is determined from the number of image data sets, wherein the repeated change in the topography of the cornea, which is caused by the cardiovascular activity, is measured, wherein, in each case, the change is measured for points (P) of the surface area of the cornea which were measured during the topography measurement, **characterised in that** the ophthalmological analysis system comprises a database which contains data of conus stages of different persons, wherein the measurement results are compared with the stored measurement results of the persons.

2. The method according to claim 1,
**characterised in that**
an amplitude of the change in the topography of the cornea (15) is measured, wherein, in each case, the amplitude is measured for the points (P) which were measured during the topography measurement.

3. The method according to claim 1 or 2,
**characterised in that**
a comparison of average values of the respective repeated changes in the points (P) is carried out.

4. The method according to any one of the preceding claims,
**characterised in that**
a comparison of the measured change in the topography of the cornea (15) in relation to a presumed change in the topography of the cornea for the respective points (P) is carried out.

5. The method according to any one of the preceding claims,
**characterised in that**
with a second analysis system, a cardiovascular activity of a person to be examined is measured, wherein, with the second analysis system, a number of measurement data sets of a cardiovascular rate in the measurement time interval is obtained, wherein the repeated change in the topography of the cornea (15) is synchronized with the measurement data of the cardiovascular rate in the measurement time interval.

6. The method according to claim 5,
**characterised in that**
a phase of the change in the topography is corrected relative to a phase of the change in the cardiovascular rate.

7. The method according to claim 5 or 6,
**characterised in that**
the second analysis system is a sphygmologic analysis system.

8. The method according to any one of the claims 5 to 7,
**characterised in that**
the systolic arterial pulse is measured.

9. The method according to any one of the claims 5 to 8,
**characterised in that**
a blood pressure change is continuously measured.

10. The method according to any one of the claims 5 to 9,
**characterised in that**
the change in the topography is measured between a beginning and an end of the systole.

11. The method according to any one of the claims 5 to 10,
**characterised in that**
the change in the topography is measured between a minimum pulse amplitude and a maximum pulse amplitude.

12. The method according to claim 10 or 11,
**characterised in that**
from the change in the topography of the cornea (15), a change in the curvature (ΔC) of the cornea is derived.

13. The method according to claim 12,
**characterised in that**
by means of the measurement time interval, an average value is calculated for the respective changes in the curvature (ΔC) of the points (P).

14. The method according to claim 12 or 13,
**characterised in that**
an increase in the change in the curvature (ΔC) is derived, wherein, from the increase, a stiffness of the cornea (15) is, in each case, determined for the measured points (P) of the surface area (24) of the cornea.

15. The method according to any one of the claims 5 to 14,
**characterised in that**
a non-invasive measurement of the cardiovascular rate is carried out.

16. The method according to any one of the preceding claims,
characterszed in that
with the ophthalmological analysis system, a measurement of a tear film is carried out from the image data sets in the measurement time interval, wherein the change in the topography is corrected corresponding to the measurement data of the tear film.

17. The method according to any one of the preceding claims,
**characterised in that**
the measurement results of the changes in the points (P) are graphically illustrated as a map of a corneal surface (14).

18. A keratometer for measuring a cornea of an eye (17) to be examined, in particular of a person, with an ophthalmological analysis system, wherein the ophthalmological analysis system serves to measure a topography of a cornea (15), wherein the ophthalmological analysis system is formed such that, in a measurement time interval, a number of image data sets of a surface area (24) of the cornea can be obtained, wherein subsequently, in the measurement time interval, a change in an intraocular pressure of the eye is effected by a cardiovascular activity, and due to said change in the intraocular pressure, a repeated change in the topography of the cornea in the measurement time interval is caused, wherein the ophthalmological analysis system comprises a processing unit, by means of which the repeated change in the topography of the cornea, which is caused by the cardiovascular activity, can be determined from the number of image data sets, wherein, by means of the processing unit, the repeated change in the topography of the cornea, which is caused by the cardiovascular activity, can be measured, and wherein, in each case, the change can be measured for points (P) of the surface area of the cornea which were measured during the topography measurement,
**characterised in that**
the ophthalmological analysis system comprises a database which contains data of conus stages of different persons, wherein the measurement results can be compared with the stored measurement results of the persons.

## Revendications

1. Procédé destiné à mesurer une cornée d'un oeil (17) à être examiné, notamment d'une personne, à l'aide d'un système d'analyse ophtalmologique d'un kératomètre, une topographie d'une cornée (15) étant mesurée à l'aide du système d'analyse ophtalmologique, plusieurs ensembles de données d'images d'une zone superficielle (24) de la cornée étant obtenus à l'aide du système d'analyse ophtalmologique pendant un intervalle de temps de mesure, une pression intraoculaire de l'oeil se changeant pendant l'intervalle de temps de mesure en raison d'une activité cardiovasculaire, ledit changement de la pression intraoculaire provoquant un changement répété de la topographie de la cornée pendant l'intervalle de temps de mesure, ledit changement dans la topographie de la cornée qui est provoqué par l'activité cardiovasculaire et qui se répète étant déterminé à partir des plusieurs ensembles de donnés d'images, ledit changement dans la topographie de la cornée qui est provoqué par l'activité cardiovasculaire et qui se répète étant mesuré, ledit changement étant mesuré chaque fois pour des points (P) de la zone superficielle de la cornée mesurés lors de la mesure de la topographie,
**caractérisé en ce que**
le système d'analyse ophtalmologique comprend une base de données qui comporte des données d'états de cône de différentes personnes, les résultats de mesure étant comparés avec les résultats de mesure sauvegardés des personnes.

2. Procédé selon la revendication 1,
**caractérisé en ce**
**qu'**une amplitude du changement dans la topographie de la cornée (15) est mesurée, l'amplitude étant mesurée chaque fois pour les points (P) mesurés lors de la mesure de la topographie.

3. Procédé selon la revendication 1 ou 2,
**caractérisé en ce que**
des valeurs moyennes des changements répétés respectifs des points (P) sont comparées.

4. Procédé selon l'une quelconque des revendications précédentes,
**caractérisé en ce que**
le changement dans la topographie de la cornée (15) mesuré est comparé par rapport à un changement dans la topographie de la cornée présupposé pour les points (P) respectifs.

5. Procédé selon l'une quelconque des revendications précédentes,
**caractérisé en ce**
**qu'**une activité cardiovasculaire d'une personne à être examinée est mesurée à l'aide d'un deuxième système d'analyse, plusieurs ensembles de données de mesure d'une fréquence cardiovasculaire étant obtenus à l'aide du deuxième système d'analyse pendant l'intervalle de temps de mesure, le changement répété de la topographie de la cornée (15) étant synchronisé avec les données de mesure de la fréquence cardiovasculaire pendant l'intervalle de temps de mesure.

6. Procédé selon la revendication 5,
**caractérisé en ce**
**qu'**une phase du changement dans la topographie est corrigée par rapport à une phase du changement de la fréquence cardiovasculaire.

7. Procédé selon la revendication 5 ou 6,
**caractérisé en ce que**
le deuxième système d'analyse est un système d'analyse sphygmologique.

8. Procédé selon l'une quelconque des revendications 5 à 7,
**caractérisé en ce que**
le pouls systolique artériel est mesuré.

9. Procédé selon l'une quelconque des revendications 5 à 8,
**caractérisé en ce**
**qu'**un changement de la pression artérielle est mesurée continûment.

10. Procédé selon l'une quelconque des revendications 5 à 9,
**caractérisé en ce que**
le changement dans la topographie est mesuré entre un début et une fin de la systole.

11. Procédé selon l'une quelconque des revendications 5 à 10,
**caractérisé en ce que**
le changement dans la topographie est mesuré entre une amplitude de pouls minimale et une amplitude de pouls maximale.

12. Procédé selon la revendication 10 ou 11,
**caractérisé en ce**
**qu'**un changement (ΔK) dans la courbure de la cornée (15) est dérivé du changement dans la topographie de la cornée.

13. Procédé selon la revendication 12,
**caractérisé en ce**
**qu'**une valeur moyenne est établie pour les changements (ΔK) dans la courbure respectifs des points (P) à travers l'intervalle de temps de mesure.

14. Procédé selon la revendication 12 ou 13,
**caractérisé en ce**
**qu'**une pente du changement (ΔK) dans la courbure est dérivée, une rigidité de la cornée (15) étant déterminée à partir de la pente pour chacun des points (P) mesurés de la zone superficielle (24) de la cornée.

15. Procédé selon l'une quelconque des revendications 5 à 14,
**caractérisé en ce que**
la fréquence cardiovasculaire est mesurée de façon non invasive.

16. Procédé selon l'une quelconque des revendications précédentes,
**caractérisé en ce que**
le système d'analyse ophtalmologique mesure le film lacrymal à partir des ensembles de données d'images pendant l'intervalle de temps de mesure, le changement dans la topographie étant corrigé selon les données de mesure du film lacrymal.

17. Procédé selon l'une quelconque des revendications précédentes,
**caractérisé en ce que**
les résultats de mesure des changements des points (P) sont affichés graphiquement comme carte d'une surface de la cornée (14).

18. Kératomètre destiné à mesurer une cornée d'un oeil (17) à être examiné, notamment d'une personne, à l'aide d'un système d'analyse ophtalmologique, ledit système d'analyse ophtalmologique servant à mesurer une topographie d'une cornée (15), ledit système d'analyse ophtalmologique étant réalisé de sorte que plusieurs ensembles de données d'images d'une zone superficielle (24) de la cornée peuvent être obtenus pendant un intervalle de temps de mesure, une pression intraoculaire de l'oeil se changeant alors pendant l'intervalle de temps de mesure en raison d'une activité cardiovasculaire, et ledit changement de la pression intraoculaire provoquant un changement répété dans la topographie de la cornée pendant l'intervalle de temps de mesure, ledit système d'analyse ophtalmologique comprenant une unité de traitement moyennant laquelle le changement dans la topographie de la cornée qui est provoqué par l'activité cardiovasculaire et qui se répète peut être déterminé à partir des plusieurs ensembles de donnés d'images, ledit changement dans la topographie de la cornée qui est provoqué par l'activité cardiovasculaire et qui se répète pouvant être mesuré moyennant ladite unité de traitement, et ledit changement pouvant être mesuré chaque fois pour des points (P) de la zone superficielle de la cornée mesurés lors de la mesure de la topographie,
**caractérisé en ce que**
le système d'analyse ophtalmologique comprend une base de données qui comporte des données d'états de cône de différentes personnes, les résultats de mesure pouvant être comparés avec les résultats de mesure sauvegardés des personnes.
